(19) Europäisches Patentamt
European Patent Office
Office européen des brevets

(11) Veröffentlichungsnummer: 0 129 829
A1

(12) # EUROPÄISCHE PATENTANMELDUNG

(21) Anmeldenummer: 84107012.1

(22) Anmeldetag: 19.06.84

(51) Int. Cl.[4]: C 07 D 401/12
C 07 D 417/12, C 07 F 9/65
A 01 N 43/40, A 01 N 43/78
A 01 N 57/32

(30) Priorität: 22.06.83 CH 3413/83
22.06.83 CH 3414/83

(43) Veröffentlichungstag der Anmeldung:
02.01.85 Patentblatt 85/1

(84) Benannte Vertragsstaaten:
BE CH DE FR IT LI NL SE

(71) Anmelder: CIBA-GEIGY AG
Postfach
CH-4002 Basel(CH)

(72) Erfinder: Gehret, Jean-Claude, Dr.
Im Aeschfeld 12
CH-4147 Aesch(CH)

(72) Erfinder: Traber, Walter, Dr.
Neueneichweg 12
CH-4153 Reinach(CH)

(74) Vertreter: Zumstein, Fritz sen., Dr. et al,
Bräuhausstrasse 4
D-8000 München 2(DE)

(54) **Schädlingsbekämpfungsmittel.**

(57) Substituierte Phenylhydrazonpyrrolidin-Derivate der Formel

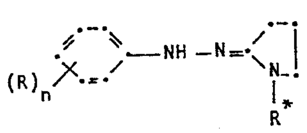

in welcher
R Halogen oder $C_1$-$C_2$-Alkyl,
n 0, 1 oder 2 und
R* die Reste $-C=N-R_1$ oder

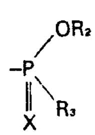

bedeuten und worin
$R_1$ einen über ein Kohlenstoffatom gebundenen unsubstituierten oder durch eine Methylgruppe substituierten Pyridinrest oder einen 2-Thiazolyrest und
$R_2$ Methyl oder Aethyl,
$R_3$ $C_1$-$C_4$-Alkyl, $C_1$-$C_2$-Alkoxy oder $C_1$-$C_4$-Alkylthio und
X Sauerstoff oder Schwefel darstellen,

besitzen wertvolle Eigenschaften zur Bekämpfung von Schädlingen wie pflanzen- und tierparasitäre Insekten und Acariden, darunter vor allem Ektoparasiten.

EP 0 129 829 A1

0123829



CIBA-GEIGY AG 5-14475/76

Basel (Schweiz)

Schädlingsbekämpfungsmittel

Die vorliegende Erfindung betrifft neue substituierte Phenylhydrazonpyrrolidin-Verbindungen, Verfahren zu ihrer Herstellung, Mittel, die diese Verbindungen als aktive Komponenten enthalten, und die Verwendung dieser Verbindungen zur Bekämpfung von Schädlingen, insbesondere von pflanzen- und tierparasitären Insekten sowie von Vertretern der Ordnung Acarina, darunter vor allem Ektoparasiten wie beispielsweise Milben und insbesondere Zecken.

In der deutschen Offenlegungsschrift Nr. 3 035 822 werden Phenylhydrazinpyrrolin-Verbindungen beschrieben und zur Bekämpfung von Milben vorgeschlagen. Diese Stoffe können jedoch die in der Praxis an sie gestellten Forderungen nur teilweise befriedigen.

Die neuen Verbindungen entsprechen der allgemeinen Formel I

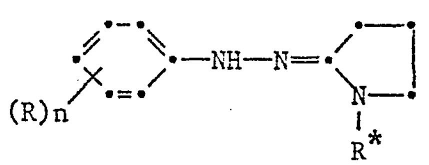

(I)

in welcher

R Halogen oder $C_1$-$C_4$-Alkyl,

n 0, 1 oder 2 und

$R^*$ die Reste -CH=N-$R_1$ oder 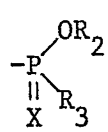 bedeuten und worin

$R_1$ einen über ein Kohlenstoffatom gebundenen unsubstituierten oder durch eine Methylgruppe substituierten Pyridinrest oder einen 2-Thiazolylrest und

$R_2$ Methyl oder Aethyl,
$R_3$ $C_1$-$C_4$-Alkyl, $C_1$-$C_2$-Alkoxy oder $C_1$-$C_4$-Alkylthio und
X Sauerstoff oder Schwefel darstellen
und schliessen die Säureadditionssalze dieser Verbindungen mit ein.

Je nach Bedeutung des Substituenten R* werden also Amidin-Derivate oder (Thio)Phosphoryl-Derivate von der Formel I umfasst.

Beispiele salzbildender Säuren sind anorganische Säuren: Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Weinsäure, Ameisensäure, Oxalsäure, Bernsteinsäure, Maleinsäure, Milchsäure, Glykolsäure, Aconitsäure, Zitronensäure, Benzoesäure, Benzolsulfonsäure, p-Toluolsulfonsäure oder Methansulfonsäure.

Das für R stehende Halogen bedeutet z.B. Fluor, Chlor, Brom oder Jod. Das als Bedeutung von $R_3$ als Substituent oder als Teilsubstituent genannte Alkyl umfasst Methyl, Aethyl und die Isomeren des Propyls und Butyls. $C_1$-$C_2$-Alkoxy stellt in seiner Bedeutung für $R_3$ Methoxy und Aethoxy dar.

Von den Verbindungen der Formel I, worin $R^*$ den Rest $-CH=N-R_1$ (Verbindungsgruppe Ia) darstellt, sind diejenigen bevorzugt, in denen R Chlor und/oder Methyl und n 1 oder 2 bedeuten, während $R_1$ die unter Formel I angegebenen Bedeutungen besitzt. Unter den bevorzugten Verbindungen sind Verbindungen, in welchen $R_{(n)}$ 2,3-Dichlor darstellt, besonders vorteilhaft.

Als Einzelverbindungen sind die weiter unten genannten Verbindungen Nr. 2a und 3a(Tabelle I) besonders bevorzugt.

Von den Verbindungen der Formel I werden die Amidine der Formel Ia

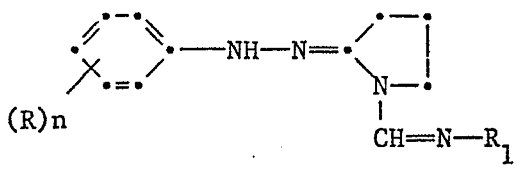

(Ia)

worin R, $R_1$ und n die unter Formel I genannte Bedeutung haben, hergestellt, indem man eine Verbindung der Formel IIa mit einer Verbindung der Formel IIIa reagieren lässt,

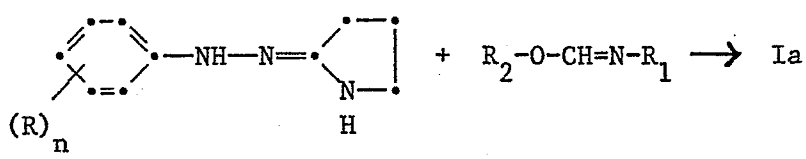


(IIa) (IIIa)

worin R, $R_1$ und n die vorgenannten Bedeutungen besitzen und $R_2$ eine Methyl- oder Aethylgruppe darstellt.

Das Verfahren wird in einem gegenüber den Reaktionsteilnehmern inerten Lösungs- oder Verdünnungsmittel bei einer Temperatur von -10° bis 100°C, vorzugsweise 10° bis 60°C, durchgeführt.

Von den Verbindungen der Formel I werden die Verbindungen der Formel Ib, worin $R^*$ den wie oben definierten Rest $-P(X)(OR_2)R_3$ bedeutet

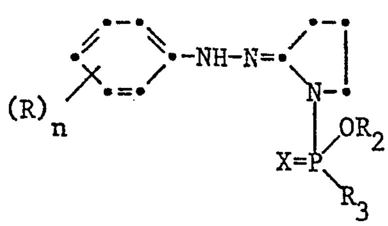

(Ib)

hergestellt, indem man eine Verbindung der Formel IIb mit einer Verbindung der Formel IIIb reagieren lässt,

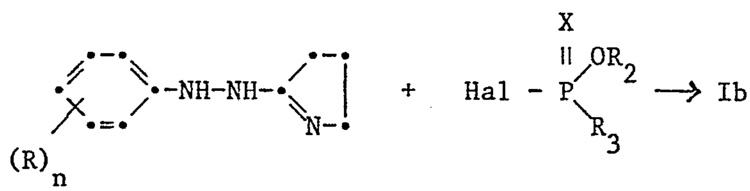

(IIb) (IIIb)

worin R, $R_2$, $R_3$, X und n die unter Formel I angegebenen Bedeutungen besitzen und Hal für ein Halogenatom, insbesondere ein Chlor- oder Bromatom, steht.

Das Verfahren wird in Gegenwart einer Base sowie eines gegenüber den Reaktionsteilnehmern inerten Lösungs- oder Verdünnungsmittels bei Temperaturen von -30° bis 100°C, vorzugsweise von -10° bis 60°C, durchgeführt.

Zur Verwendung in den vorgenannten Verfahren sind als Lösungs- oder Verdünnungsmittel beispielsweise folgende geeignet: Aether und ätherartige Verbindungen wie Diäthyläther, Diisopropyläther, Dioxan und Tetrahydrofuran, ferner aromatische Kohlenwasserstoffe wie Benzol, Toluol und die Xylole sowie Ketone wie Aceton, Methyläthylketon und Cyclohexanon. Ausserdem können dafür Acetonitril sowie chlorierte Kohlenwasserstoffe wie Dichlormethan, Methylenchlorid, Tetrachlorkohlenstoff oder Chlorbenzol verwendet werden.

Als Basen können z.B. eingesetzt werden:
Alkalihydroxide wie Natrium- oder Kaliumhydroxid oder Alkylamine wie Triäthylamin oder Diisopropyläthylamin sowie ferner Pyridin oder N-Methylpyrrolidon.

Die auf diese Weise hergestellten Verbindungen der Formel I können nach an sich bekannten Methoden in ihre Säuresalze überführt werden.

Die erfindungsgemässen Verbindungen der Formel I oder die sie als aktive Komponente enthaltenden Mittel zeichnen sich überraschenderweise in der Schädlingsbekämpfung durch besonders gute biologische Aktivität und ein günstiges Wirkungsspektrum aus. Darüber hinaus besitzen sie eine unerwartet hohe Stabilität, wie sie beispielsweise bei der Verwendung in wässrigen und verschmutzten Vieh-Dips immer wieder gefordert wird. Gegen Warmblüter besitzen Verbindungen der Formel I und besonders die der Formel Ia eine überraschend hohe Verträglichkeit, die ihre Handhabung in der Praxis ausserordentlich erleichtert.

Die Aktivität der Wirkstoffe richtet sich sowohl gegen pflanzenparasitäre als auch gegen tierparasitäre Schädlinge. Dazu zählen als Pflanzenschädlinge Insekten der Ordnung: Lepidoptera, Coleoptera, Homoptera, Heteroptera, Diptera, Thysanophtera, Anoplura, Siphonaptera, Mallophaga, Thysanura, Isoptera, Psocoptera und Hymenoptera.

Die Verbindungen der Formel I sind weiterhin geeignet zur Bekämpfung von Vertretern der Ordnung Akarina der Familien: Ioxoididae, Argasidae, Tetranychidae und Dermanyssidae. Die Verbindungen der Formel I können mit Erfolg vor allem zur Bekämpfung von phytopathogenen Milben, z.B. der Familien Tetranychidae und Phytoptipalpidae (Spinnmilben), Tarsonemidae (Fadenfussmilben) und Eriophydiae (Gallmilben), eingesetzt werden.

Neben ihrer Wirkung gegenüber Mücken und Fliegen, wie z.B. Aëdes aegypti und Musca domestica, können Verbindungen der Formel I auch zur Bekämpfung von pflanzenschädigenden Frassinsekten in Zier- und Nutzpflanzungen, insbesondere in Baumwollkulturen (z.B.

gegen Spodoptera littoralis und Heliothis virescens) sowie in Getreide-, Obst- und Gemüsekulturen (z.B. gegen Laspeyresia pomonella, Laptinotarsa decemlineata und Epilachna varivestis) eingesetzt werden. Die Verbindungen der Formel I zeichnen sich auch durch eine gute Wirkung gegen larvale Insektenstadien und Nymphen, insbesondere von fressenden Schadinsekten, aus. Die erfindungsgemässen Verbindungen eignen sich weiterhin zur Bekämpfung von Bodeninsekten (z.B. Aulacophora femoralis, Chortophila brassicae, Diabrotica balteata, Pachnoda savigni und Scotia ypsilon).

Insbesondere können die Verbindungen der Formel I mit gutem Erfolg gegen pflanzenschädigende Zikaden, speziell in Reis-Kulturen, eingesetzt werden, wobei sie sowohl systemische als auch Kontakt-Wirkung zeigen, beispielsweise gegen Nilaparvata lugens und Laodelphax striatellus.

Die Verbindungen sind besonders gut wirksam gegen Nutztierparasitäre Zecken (Ixodidae), darunter vorzugsweise gegen die Species Rhipicephalus, Amblyomma und Boophilus, sowie gegen Milben wie beispielsweise Dermanyssus gallinae und Räudemilben wie z.B. Psoroptes ovis,Psoroptes bovis und Chorioptes bovis.

Die Wirksamkeit der Verbindungen der Formel I richtet sich ferner gegen weitere Ektoparasiten der Ordnung Diptera und deren Familien Culicidae, Simuliidae, Tipulidae, Muscidae und Calliphoridae. Dabei eignen sich die Wirkstoffe besonders gut zur Bekämpfung von Calliphoridae wie beispielsweise Lucilia sericata (Blowfly) und Lucilia cuprina.

Weiterhin gut bekämpfbar mit den Verbindungen der Formel I sind Vertreter der Ordnungen Aphaniptera (z.B. blutsaugende Flöhe) und Phthiraptera (z.B. blutsaugende Läuse).

In ihrer Aktivität zeigen die erfindungsgemässen Verbindungen eine Wirkungsbreite, die alle Entwicklungsstadien der Parasiten sowie darüber hinaus auch die Ablage fertiler Eier umfasst.

Die Bekämpfung der genannten Parasiten erfolgt beispielsweise an folgenden Haus- und Nutztieren: Rinder, Schafe, Pferde, Rotwild, Hühner, Ziegen, Katzen und Hunde.

Die erhöhte Stabilität der erfindungsgemässen Verbindungen stellt eine Wirkungsdauer sicher, die zeitlich die Entwicklungszyklen mehrerer Parasitengenerationen umfasst, so dass je nach Anwendungsform, beispielsweise bei Nutztieren, eine einzige Behandlung pro Saison ausreichend ist.

Die Verbindungen der Formel I zeigen aufgrund ihren Eigenschaften eine gute Eignung für die Bekämpfung von ektoparasitären Akariden und Insekten, z.B. durch direkte Tier- oder durch Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Harnstoffe, Carbamate oder chlorierte Kohlenwasserstoffe.

Zur Bekämpfung der Schädlinge werden die erfindungsgemässen Verbindungen der Formel I sowohl allein als auch in Form von Mitteln eingesetzt, welche noch zusätzlich geeignete Trägerstoffe und/oder Zuschlagstoffe oder Gemische solcher Stoffe enthalten. Geeignete Träger- und Formulierungshilfsstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs- oder Bindemitteln.

Die Formulierungen, d.h. die den Wirkstoff der Formel I, bzw. Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische, oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Weiterhin sind auch die Fettsäure-methyl-taurinsalze sowie modifizierte und nicht-modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf.

Als Beispiele nichtionischer Tenside seien Nonylphenolpolyäthoxyäthanole, Ricinusölpolyglykoläther, Polypropylenpolyäthylenoxydaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbiten wie das Polyoxyäthylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tensiden sind u.a. in folgender Publikation beschrieben:

"Mc Cutcheon's Detergents and Emulsifiers Annual" MC Publishing Corp., Ringwood, New Jersey, 1982.

Die pestiziden Zubereitungen enthalten in der Regel 0,1 bis 99 %, insbesondere 0,1 bis 95 %, Wirkstoff der Formel I oder Kombinationen dieser Wirkstoffe mit anderen Insektiziden oder Akariziden, 1 bis 99,9 % eines festen oder flüssigen Zusatzstoffes und 0 bis 25 %, insbesondere 0,1 bis 20 %, eines Tensides. Während als Handelware eher konzentrierte Mittel bevorzugt werden, verwendet der Endverbraucher in der Regel verdünnte Mittel von 0,001-1% Wirkstoffgehalt.

Die Herstellung der erfindungsgemässen Mittel erfolgt an sich bekannter Weise durch inniges Vermischen und/oder Vermahlen von Wirksstoffen der Formel I mit geeigneten Trägerstoffen, gegebenenfalls unter Zusatz von gegenüber den Wirkstoffen inerten Dispergier- und Lösungsmitteln.

Die Mittel können auch weitere Zusätze, wie Stabilisatoren, Entschäumer, Viskositätsregulatoren, Bindemittel, Haftmittel, Phospholipide oder andere Wirkstoffe zur Erzielung spezieller Effekte enthalten.

Die Applikation der Verbindungen der Formel I oder die sie enthaltenden Mittel wird je nach Anwendungszweck und -ziel beispielsweise durch Versprühen, z.B. in Sprühgängen (spray races), durch Tauchen, z.B. in Viehbädern (cattle dips), durch Aufgiessen z.B. im pour-on-Verfahren, durch Waschen z.B. im Tier-Dressing-Verfahren sowie ferner durch Vernebeln, Verstäuben, Verstreuen oder durch Giessen durchgeführt.

Beispiel 1:

Herstellung von N-(2-[N'-2',3'-Dichlorphenylhydrazono)-pyrrolidin-1-yl]-methyliden-N-(6-methylpyridin-2-yl)-amin [Verb. 1a]

Zu 98 g (0,4 M) 2-[N'(2',3'-Dichlorphenylhydrazino)-pyrrolin, gelöst in 900 ml Toluol, wird bei ca. 25°C eine Lösung von 65,6 g (0,4 M) N-(6-Methylpyridin-2-yl)-formiminäthyläther in 100 ml Toluol langsam unter kräftigem Rühren zugetropft. Das Reaktionsgemisch wird anschliessend 48 Stunden bei ca. 45°C bis zum Abschluss der Reaktion gerührt, dann filtriert und das Filtrat eingeengt. Nach Zugabe von ca. 300 ml Diäthyläther kristallisiert ein gelbes Endprodukt aus.
Ausbeute: 100 g (= 68 % d.Th.) Schmelzpunkt: 150-152°C.

Beispiel 2:

Herstellung von N-(2-[N'-(2',3'-Dichlorphenylhydrazono)-pyrrolidin-1-yl)]-methyliden-N-(3-methylpyridin-2-yl)-amin [Verb. 2a]

Zu 9.8 g (0.04 M) 2-N'(2',3'-Dichlorphenylhydrazino)-pyrrolin, gelöst in 90 ml Toluol, wird bei 20-26°C eine Lösung von 6.6 g (0.04 M) N-(3-Methylpyridin-2-yl)-formiminäthyläther in 60 ml Toluol langsam unter kräftigem Rühren zugetropft. Das Reaktionsgemisch wird anschliessend 12 Stunden bei ca. 45°C bis zum Abschluss der Reaktion gerührt, dann filtriert und das Filtrat eingeengt. Nach Zugabe von ca. 50 ml Diäthyläther kristallisiert ein gelbes Endprodukt aus.
Ausbeute: 7.3 g (= 50.3 % d. Th.) Schmelzpunkt: 155-158°C.

Beispiel 3:

Herstellung von 1-(O-Aethyl-S-n-propyl-thiophosphinyl)-2-[N'-(2',3'-dichlorphenylhydrazono)]-pyrrolidin [Verb. 1b]

Zu 14,6 g (0,06 M) 2-[N'-(2',3'-Dichlorphenylhydrazino)]-pyrrolin, gelöst in 50 ml Tetrahydrofuran, werden 7,6 g (0,075 M) Triäthylamin in 80 ml Toluol gegeben. Dann wird unter ständigem Rühren bei 0-5°C langsam eine Lösung von 13,1 g (0,06 M) O-Aethyl-S-n-propylthiochlorphosphat in 80 ml Toluol zugetropft. Anschliessend wird 24 Stunden bei ca. 50°C bis zum Reaktionsende gerührt, dann filtriert, das Filtrat eingeengt und über Aluminiumoxid mit einem Diäthyläther/Hexangemisch (1:1 v/v) chromatographiert. Es wird ein farbloses Endprodukt erhalten.

Ausbeute: 11,5 g (= 45 % d.Th.) Schmelzpunkt: 91-93°C.

Analog den vorstehend beschriebenen Beispielen werden folgende Verbindungen der Formel I hergestellt:

Tabelle 1

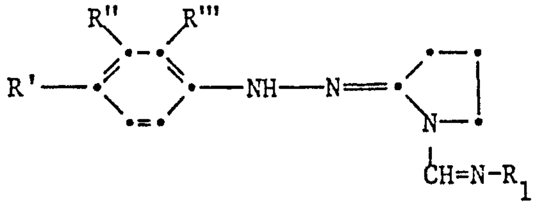

| Nr | R' | R'' | R''' | $R_1$ | Fp. [°C] |
|---|---|---|---|---|---|
| 1 a | H | Cl | Cl | 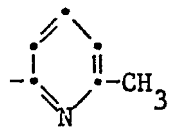 | 150 - 152 |
| 2 a | H | Cl | Cl | 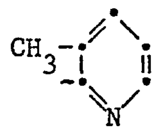 | 155 -158 |
| 3 a | H | Cl | Cl | 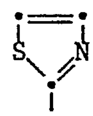 | 137 - 139 |
| 4 a | H | H | Cl | 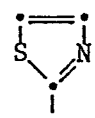 | 122 - 124 |
| 5 a | Cl | Cl | H | 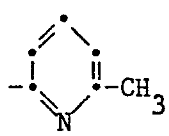 | 167 - 169 |
| 6 a | Cl | Cl | H | 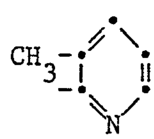 | 179 - 180 |
| 7 a | H | Cl | Cl | 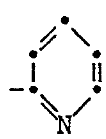 | 164-167 |
| 8 a | H | Cl | Cl | 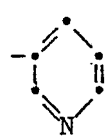 | |
| 9 a | H | Cl | Cl | 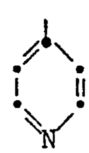 | |

Tabelle II

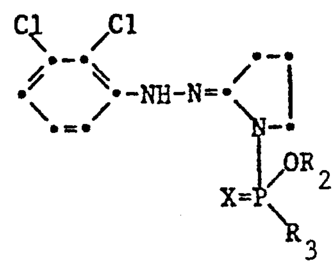

| Nr. | $R_2$ | $R_3$ | X | Fp. [°C] |
|---|---|---|---|---|
| 1b | $C_2H_5$ | $SC_3H_7(n)$ | S | 91-93 |
| 2b | $C_2H_5$ | $C_2H_5$ | S | 79-81 |
| 3b | $C_2H_5$ | $SC_4H_9(i)$ | O | |
| 4b | $C_2H_5$ | $OC_2H_5$ | O | |
| 5b | $CH_3$ | $OCH_3$ | O | |
| 6b | $C_2H_5$ | $SC_3H_7(n)$ | O | |

Beispiel 4:

Wirkung gegen Zecken: Abtötungswirkung in verschiedenen Entwicklungsstadien

Als Testobjekte werden Larven (jeweils ca. 50) oder Nymphen (jeweils ca. 25) der Zeckenarten Amblyomma hebraeum und Boophilus microplus verwendet. Die Testorganismen werden für kurze Zeit in wässrige Emulsionen bzw. Lösungen der Salze der zu untersuchenden Substanzen von bestimmter Konzentration getaucht. Die in den Teströhrchen befindlichen Emulsionen bzw. Lösungen werden dann mit Watte aufgenommen und die benetzten Testtiere in den so kontaminierten Röhrchen belassen. Die Auswertung erfolgt für Larven nach 3 Tagen und für Nymphen und Imagines nach 14 Tagen. Es wird die minimale Substanzkonzentration ermittelt, die die 100%ige Abtötung bewirkt, ausgedrückt im ppm Wirksubstanz bezogen auf das Total von Emulsion oder Lösung.

Die Verbindungen der Tabellen I und II zeigen im vorstehenden Test vollständige Abtötung bei Anwendungskonzentrationen von 25 ppm, die Verbindungen Nr. 1a, 2a, 3a und 6a sowie die Verbindungen Nr. 1b und 2b bereits bei 12,5 ppm. Diese Aktivität bleibt auch noch bei einer mehrere Wochen alten Lösung der Verbindung Nr. 2a oder Nr. 3a bestehen.

Beispiel 5: Wirkung gegen Zecken: Eiablagehemmung

Als Testtiere werden vollgesogene Weibchen der Rinderzecke Boophilus microplus verwendet. Pro Konzentration werden je 10 Zecken eines OPresistenten Stammes (z.B. Biarra-Stamm) und eines normal empfindlichen Stammes (z.B. Yeerongpilly-Stamm) behandelt. Die Zecken werden auf mit Doppelklebeband bezogenen Platten fixiert und dann entweder mit wässrigen Emulsionen bzw. mit Lösungen der Salze der zu untersuchenden Verbindungen benetzt oder mit einem mit diesen Flüssigkeiten getränkten Wattebausch in Berührung gebracht. Anschliessend werden sie in einem klimatisierten Raum bei konstanten Bedingungen aufbewahrt. Die Auswertung erfolgt nach drei Wochen. Es wird die totale Hemmung der Ablage von fertilen Eiern ermittelt.

Die Hemmwirkung der Substanzen wird ausgedrückt als minimale Substanzkonzentration in ppm bei 100%-iger Wirkung gegen normal sensible und resistente adulte weibliche Zecken.

Die Verbindungen der Tabellen I und II weisen im vorstehenden Test vollständige Abtötung bei 25 ppm auf.

Beispiel 6: Wirkung gegen pflanzenparasitäre Insekten

Baumwollpflanzen werden mit einer Versuchslösung, enthaltend 25, 50 oder 100 ppm der zu prüfenden Verbindung, besprüht.

Nach dem Antrocknen des feuchten Belages werden die Baumwollpflanzen mit Spodoptera littoralis-Larven ($L_3$) besetzt. Der Versuch wird bei 24°C und 60 % relativer Luftfeuchtigkeit durchgeführt.

Die Verbindungen der Tabellen I und II zeigen gute Frassgift-Wirkung gegen Spodoptera-Larven, wobei die Verbindungen Nr. 1a-3a bei 50 ppm Anwendungskonzentration vollständige Abtötung und die Verbindungen der Tabelle II bei 25 ppm Anwendungskonzentration eine über 90%-ige Abtötung erzielen.

Beispiel 7: Wirkung gegen Lucilia sericata ($L_1$)

Als Testorganismen werden frisch geschlüpfte Larven 1 verwendet. 1 ml einer wässrigen Suspension bzw. Lösung der Aktivsubstanz mit einem Wirkstoffgehalt von 1000 ppm wird mit 3 ml eines speziellen Larvenzuchtmediums bei 50°C vermischt, so dass eine homogene Mischung von 250 ppm Wirkstoffgehalt entsteht. Pro Probe werden ca. 30 Lucilia-Larven eingesetzt. Nach vier Tagen wird die Mortalität bestimmt.

Die Verbindungen der Tabelle I zeigen eine über 90%-ige und die Verbindungen der Tabelle II zeigen eine über 80%-ige Abtötungsrate. Die Verbindungen 1, 2 und 3 der Tabelle I erzielen vollständige Abtötung.

Beispiel 8: Wirkung gegen Nilaparvata lugens (Nymphen)

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend 100, 200, 300 oder 400 ppm Wirkstoff besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein Glaszylinder gestülpt und dieser mit einem Gaze-Deckel abgedeckt. Die Nymphen werden bis zum

Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Tabellen I und II zeigen im vorstehenden Test gute Wirkung. So bewirken die Verbindung Nr.1a bei 100 ppm Anwendungskonzentration und die Nr. 2a bei 400 ppm Anwendungskonzentration jeweils 80 % Abtötung.

Beispiel 9: Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in steigenden Konzentrationen von 3 ppm bis 400 ppm enthalten. Dann werden Plastikbecher, die einen oberen Durchmesser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Ansatz wird die in den Bechern enthaltene Erde abgesiebt und die zurückbleibenden Larven hinsichtlich ihrer Mortalität geprüft.

Verbindungen der Tabellen I und II zeigen in diesem Test gute Wirksamkeit, wobei die Verbindungen Nr. 1a bei 12,5 ppm Anwendungskonzentration und Nr. 2a bei 400 ppm Anwendungskonzentration über 80 % Abtötung bewirken.

Aehnliche Resultate, wie in den vorstehenden Beispielen 3 bis 9 angegeben, können mit Wirkstoffen aus den Tabellen I und II auch bei längerer Anwendungszeit unter Praxisbedingungen ohne Durchführung zusätzlicher pH-Wert-stabilisierender Massnahmen in den praxisbezogenen Anwendungsformen erzielt werden.

Beispiel 10: Emulsionskonzentrat

20 Gew.-Teile Wirkstoff werden in
70 Gew.-Teilen Xylol gelöst und mit
10 Gew.-Teilen eines Emulgiermittels, bestehend aus einem Gemisch von Tributylphenylpolyäthylenglykoläther und dem Calciumsalz der Dodecylbenzolsulfonsäure, versetzt. Das Emulsionskonzentrat kann in beliebigem Verhältnis mit Wasser versetzt werden und bildet dabei eine milchige Emulsion.

Beispiel 11: Spritzpulver

5 bis 30 Gew.-Teile Wirkstoff werden in einer Mischapparatur mit
5 Gew.-Teilen eines aufsaugenden Trägermaterials (z.B. hochdisperse Kieselsäure) und
55 bis 80 Gew.-Teilen eines Trägermaterials [Bolus alba oder Kaolin und einem Dispergiermittelgemisch, bestehend aus
5 Gew.-Teilen von Na-Laurylsulfonats und
5 Gew.-Teilen von Octylphenolpolyäthylenglykol intensiv vermischt. Diese Mischung wird in einer Stift- oder Luftstrahlmühle bis auf 5-15 µm gemahlen. Das so erhaltene Spritzpulver gibt in Wasser eine gute Suspension.

Beispiel 12: Stäubemittel

5 Gew.-Teile feingemahlener Wirkstoff werden mit
2 Gew.-Teilen einer gefällten Kieselsäure und
93 Gew.-Teilen Talk intensiv gemischt.

Beispiel 13: Aufguss-Lösung (Pour on)

| | |
|---|---|
| Wirksubstanz | 30,0 g |
| Natrium-dioctylsulfosuccinat | 3,0 g |
| Benzylalkohol | 48,0 g |
| Erdnussöl | 19,8 g |
| | 100,8 g = 100 ml |

Die Wirksubstanz wird in dem Benzylalkohol unter Rühren, eventuell auch unter leichtem Erwärmen, gelöst. Zu der Lösung wird das Natriumdioctylsulfosuccinat und das Erdnussöl gegeben und unter Erwärmen und gründlichem Durchmischen gelöst.

Beispiel 14: Wasch-Lösung (dressing)

| | |
|---|---|
| Wirksubstanz | 30,00 g |
| Natrium-Dioctylsulfosuccinat | 3,00 g |
| Benzylalkohol | 35,46 g |
| Aethylenglylkol-monomethyläther | 35,46 g |
| | 103,92 g = 100 ml |

Die Wirksubstanz wird in dem grössten Teil des Gemisches der beiden Lösungsmittel unter kräftigem Rühren gelöst. Anschliessend wird das Natrium-dioctylsulfosuccinat, eventuell unter Erwärmen, gelöst und schliesslich mit dem restlichen Lösungsmittelgemisch aufgefüllt.

Patentansprüche

1. Substituierte Phenylhydrazonpyrrolidin-Verbindungen der allgemeinen Formel I

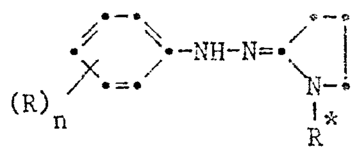

(I),

in welcher

R Halogen oder $C_1$-$C_4$-Alkyl,

n 0, 1 oder 2 und

$R^*$ die Reste $-CH{=}N{-}R_1$ oder 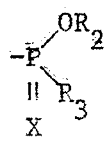

bedeuten und worin

$R_1$ einen über ein Kohlenstoff gebundenen unsubstituierten oder durch eine Methylgruppe substituierten Pyridinrest oder einen 2-Thiazolylrest und

$R_2$ Methyl oder Aethyl,

$R_3$ $C_1$-$C_4$-Alkyl, $C_1$-$C_2$-Alkoxy oder $C_1$-$C_4$-Alkylthio und

X Sauerstoff oder Schwefel darstellen,

einschliesslich der Säureadditionssalze der Verbindungen der Formel I.

2. Verbindungen der Formel I gemäss Anspruch 1, in welchem $R^*$ den Rest $-CH{=}N{-}R_1$ bedeutet und R, $R_1$ und n die in Anspruch 1 angegebenen Bedeutungen besitzen.

3. Verbindungen der Formel I gemäss Anspruch 1, in welchem $R^*$ den Rest 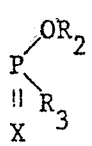

bedeutet, und R, $R_2$, $R_3$, X und n die in Anspruch 1 angegebenen Bedeutungen besitzt.

4. Verbindungen der Formel I gemäss Anspruch 2, in welchem R Chlor und/oder Methyl und n 1 oder 2 bedeuten und $R_1$ die unter Formel I angegebene Bedeutung besitzt.

5. Verbindungen der Formel I gemäss Anspruch 4, worin $(R)_n$ 2,3-Dichlor darstellt.

6. N-(2-N'-(2',3'-Dichlorphenylhydrazono)-pyrrolidin-1-yl]-methyliden)-N-(6-methylpyridin-2-yl)-amin.

7. N-(2-[N'-(2',3'-Dichlorphenylhydrazono)-pyrrolidin-1-yl]-methyliden)-N-(3-methylpyridin-2-yl)-amin.

8. N-(2-[N'-(2',3'-Dichlorphenylhydrazono)-pyrrolidin-1-yl]-methyliden)-N-thiazol-2-yl)-amin.

9. Verfahren zur Herstellung von Verbindungen der Formel Ia

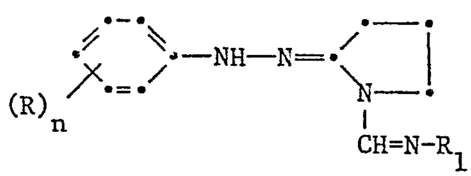

(Ia)

in welcher

R Halogen oder $C_1$-$C_4$-Alkyl und

$R_1$ einen über ein Kohlenstoffatom gebundenen unsubstituierten oder durch eine Methylgruppe substituierten Pyridinrest oder einen 2-Thiazolylrest darstellen und

n 0,1 oder 2 bedeutet,

dadurch gekennzeichnet, dass man eine Verbindung der Formel IIa

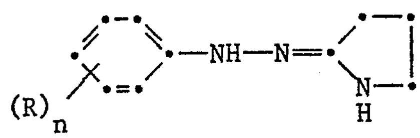

(IIa)

mit einer Verbindung der Formel IIIa

$$R_2\text{-O-CH=N-}R_1$$

(IIIa),

in welchen
R, $R_1$ und n die unter Formel Ia angegebenen Bedeutungen besitzt und $R_2$ Methyl oder Aethyl darstellen in Gegenwart eines Lösungs- oder Verdünnungsmittels bei Temperaturen von -10° bis 100°C reagieren lässt.

10. Verfahren gemäss Anspruch 9, dadurch gekennzeichnet, dass die Reaktionstemperatur 10° bis 60°C beträgt.

11. Schädlingsbekämpfungsmittel, dadurch gekennzeichnet, dass es als aktive Komponente mindestens eine Verbindung der Formel I gemäss Anspruch 1 neben inerten Träger- und Formulierungshilfsstoffen enthält.

12. Schädlingsbekämpfungsmittel gemäss Anspruch 11, dadurch gekennzeichnet, dass es als aktive Komponente mindestens eine Verbindung gemäss den Ansprüchen 2 bis 8 enthält.

13. Verfahren zur Bekämpfung von Schädlingen, dadurch gekennzeichnet, dass eine Verbindung der Formel I gemäss Anspruch 1 verwendet wird.

14. Verfahren gemäss Anspruch 13, wobei die Schädlinge Ektoparasiten sind.

15. Verfahren gemäss Anspruch 13, wobei die Schädlinge Insekten oder Vertreter der Ordnung Acarina sind.

16. Verwendung der Verbindungen gemäss den Ansprüche 1 bis 8 zur Bekämpfung von Schädlingen.

17. Verwendung gemäss Anspruch 16 zur Bekämpfung von Ektoparasiten.

18. Verwendung gemäss Anspruch 16 zur Bekämpfung von Insekten und Vertreter der Ordnung Acarina.

FO 7.5 HGZ/hc*/rn*

0129829

Europäisches Patentamt

# EUROPÄISCHER RECHERCHENBERICHT

Nummer der Anmeldung

EP 84107012.1

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| D,A | DE - A1 - 3 035 822 (TAKEDA)<br>* Ansprüche 1,8 *<br>-- | 1,11,<br>13 | C 07 D 401/12<br>C 07 D 417/12<br>C 07 F 9/65<br>A 01 N 43/40<br>A 01 N 43/78<br>A 01 N 57/32 |
| A | DE - A - 2 235 113 (BAYER)<br>* Ansprüche 1,2,3 *<br>-- | 1,11,<br>13,16 | |
| A | DE - A - 2 109 577 (BOEHRINGER)<br>* Seite 1; Seite 2, Zeilen 1-10; Ansprüche 13,23,56 *<br>---- | 1 | |
| | | | RECHERCHIERTE SACHGEBIETE (Int. Cl. 3) |
| | | | C 07 D 401/00<br>C 07 D 417/00<br>C 07 F |

Der vorliegende Recherchenbericht wurde für alle Patentansprüche erstellt.

| Recherchenort | Abschlußdatum der Recherche | Prüfer |
|---|---|---|
| WIEN | 05-09-1984 | HOCHHAUSER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument